(19) Europäisches Patentamt
European Patent Office
Office européen des brevets

(11) Veröffentlichungsnummer: **0 502 492 A2**

(12) **EUROPÄISCHE PATENTANMELDUNG**

(21) Anmeldenummer: **92103664.6**

(22) Anmeldetag: **04.03.92**

(51) Int. Cl.[5]: **C07C 49/84**, C07C 205/45, C07C 323/22, C07C 317/24, C07C 255/56, C07C 45/54, A01N 35/06

(30) Priorität: **06.03.91 DE 4107141**

(43) Veröffentlichungstag der Anmeldung: **09.09.92 Patentblatt 92/37**

(84) Benannte Vertragsstaaten: **AT BE CH DE ES FR GB IT LI NL**

(71) Anmelder: **HOECHST AKTIENGESELLSCHAFT**
**Postfach 80 03 20**
**W-6230 Frankfurt am Main 80(DE)**

(72) Erfinder: **Stark, Herbert, Dr.**
**Gimbacher Tann 15**
**W-6233 Kelkheim (Taunus)(DE)**
Erfinder: **Bauer, Klaus, Dr.**
**Doorner Strasse 53d**
**W-6450 Hanau 7(DE)**
Erfinder: **Bieringer, Hermann, Dr.**
**Eichenweg 26**
**W-6239 Eppstein/Taunus(DE)**

(54) **Haloalkoxy-substituierte Benzoylcyclohexandione als Herbizide und Pflanzenwachstumsregulatoren.**

(57) Verbindungen der Formel I

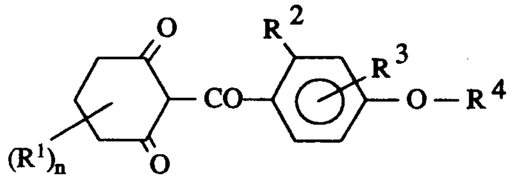

(I)

worin
n 0-6, $R^1$ Alkyl, Haloalkyl, Cycloalkyl, Halocycloalkyl, Phenyl oder halogeniertes Phenyl,
$R^2$ Hal, CN, Alkyl, Haloalkyl, Alkoxy, Haloalkoxy, Thioalkyl, $R^aSO_2$-, $R^aSO_2O$-, $R^aSO_2NR^6$, wobei $R^a$, $R^6$ Alkyl oder Haloalkyl sind, und $R^3$ H, Hal, Alkyl, Haloalkyl, Alkoxy, Haloalkoxy oder Alkylthio sowie $R^4$ Haloalkyl bedeuten, sind als selektive Herbizide und als Wachstumsregulatoren geeignet. Zur Herstellung kann man ein Cyclohexandion a) mit einem 4-oxy-substituierten Benzoylchlorid umsetzen und den erhaltenen Enolether umlagern oder b) mit einem Benzoylcyanid umsetzen. Das benötigte Benzoylchlorid bzw. -cyanid ist über die Carbonsäure aus entsprechend substituierten Toluolen zugänglich.

EP 0 502 492 A2

Es ist bekannt, daß einige benzoylierte Cyclohexandione herbizide und pflanzentumswachstumsregulierende Eigenschaften besitzen; vergleiche z.B. US-A-4780127, EP-A-278742 (ZA-A-88/911), EP-A-268795 (US-A-4,960,841), EP-A-264859 (US-A-4,781,751), EP-A-264737 (US-A-4,838,932), EP-A-135191 (US-A-4,816,066), EP-A-137963 (US-A-4,780,127), EP-A-186118 (US-A-4,946,981), EP-A-186119 (AU-A-85/51337), EP-A-90262 (CA-A-1217204) und EP-A-186120 (US-A-4,806,146). Die Wirkungen dieser Verbindungen sind jedoch nicht immer befriedigend.

Es wurde nun überraschend gefunden, daß einige benzoylierte Cyclohexandione mit halogenierten Alkoxy-Resten in 4-Position am Phenylring besonders gut als Herbizide und Pflanzenwachstumsregulatoren geeignet sind.

Gegenstand der vorliegenden Erfindung sind daher Verbindungen der Formel (I) oder deren Salze,

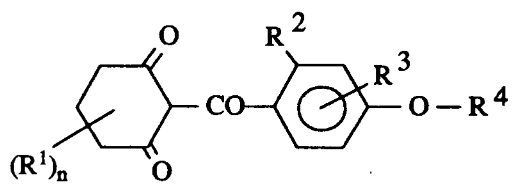

(I)

worin

- n eine ganze Zahl von 0 bis 6,
- $R^1$ gleiche oder verschiedene Substituenten aus der Gruppe gradkettiges $C_1$-$C_4$-Alkyl, verzweigtes $C_3$-$C_4$-Alkyl, $C_3$-$C_6$-Cycloalkyl und Phenyl, wobei die genannten Reste unsubstituiert oder durch ein oder mehrere Halogen-Atome substituiert sind,
- $R^2$ Halogen, $NO_2$, CN, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Thioalkyl, $R^aSO_2$-, $R^aSO_2$-O-, $R^aSO_2NR^b$-, wobei $R^a$ und $R^b$ unabhängig voneinander $C_1$-$C_2$-Alkyl oder $C_1$-$C_2$-Halogenalkyl bedeuten,
- $R^3$ Wasserstoff, Halogen, $C_1$-$C_3$-Alkyl, $C_1$-$C_3$-Halogenalkyl, $C_1$-$C_3$-Alkoxy, $C_1$-$C_3$-Halogenalkoxy oder $C_1$-$C_3$-Alkylthio und
- $R^4$ $C_1$-$C_3$-Halogenalkyl bedeuten.

In der Formel (I) können Alkyl-, Alkoxy-, Haloalkyl-, Alkylamino- und Alkylthioreste sowie die entsprechenden ungesättigten und/oder substiuierten Reste, wenn nicht anders angegeben, jeweils geradkettig oder verzweigt sein. Alkylreste, auch in den zusammengesetzten Bedeutungen wie Alkoxy, Haloalkyl usw., bedeuten beispielsweise Methyl, Ethyl n- oder i-Propyl, n-, i-, t- oder 2-Butyl; Alkenyl- und Alkinylreste haben die Bedeutung der den Alkylresten entsprechenden möglichen ungesättigten Reste, wie 2-Propenyl, 2- oder 3-Butenyl, 2-Propinyl, 2- oder 3-Butinyl. Halogen bedeutet Fluor, Chlor, Brom oder Iod.

Abhängig von Stellung und Anzahl der Reste $R^1$ können Verbindungen der Formel (I) stereoisomer sein. Die Erfindung betrifft somit alle Stereoisomeren, die durch die allgemeine Formel (I) umfaßt sind, und Gemische derselben.

Die erfindungsgemäßen Verbindungen der Formel (I) können aufgrund von Tautomerie-Gleichgewichten folgende 3 Strukturen haben:

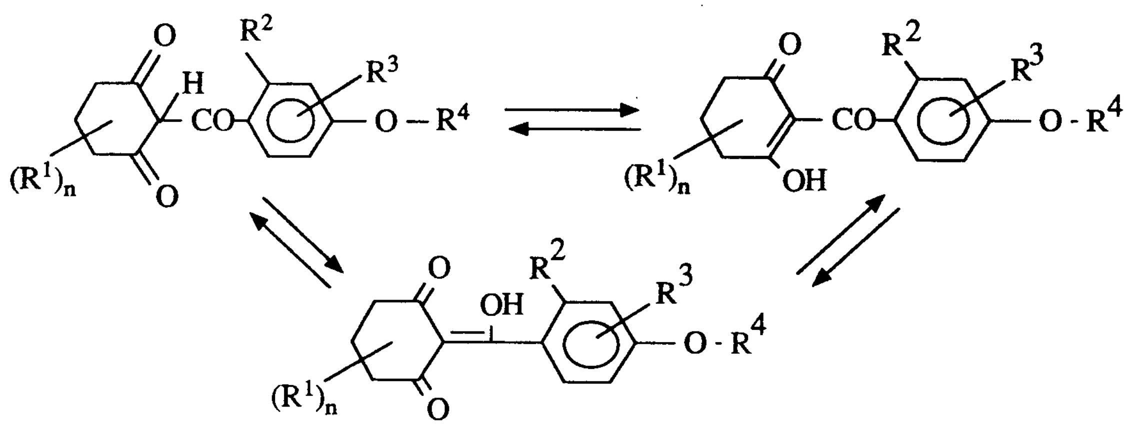

Darin haben n, $R^1$, $R^2$, $R^3$ und $R^4$ die oben beschriebene Bedeutung.

Das bewegliche Proton der tautomeren Strukturen ist sauer und kann durch Reaktion mit einer Base entfernt werden. Das Anion des dabei gebildeten Salzes kann dabei die folgenden Resonanzstrukturen haben:

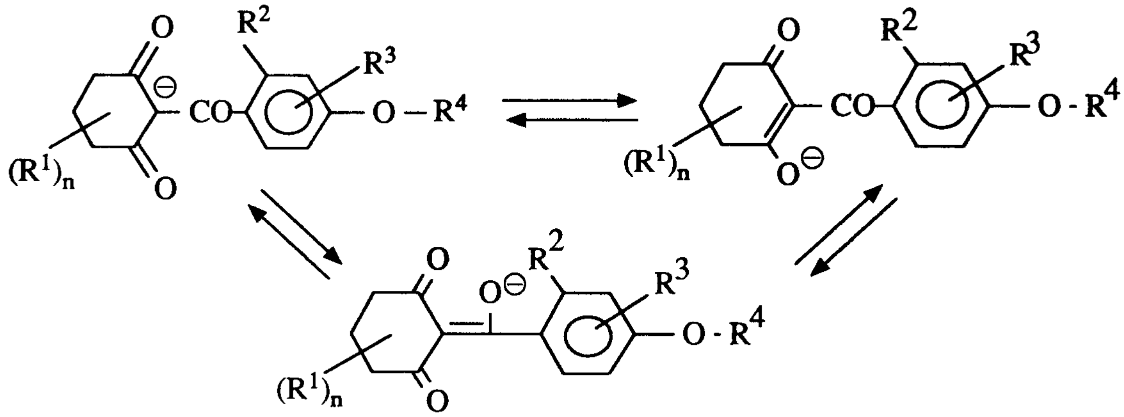

Dabei haben n, $R^1$, $R^2$, $R^3$ und $R^4$ die oben beschriebene Bedeutung.

Als Gegenionen für diese Anionen kommen beispielsweise anorganische Kationen wie die Kationen der Alkalimetalle, z.B. Lithium, Natrium und Kalium, oder Erdalkalimetalle, z.B. Kalzium und Magnesium, oder Ammonium oder organische Kationen, wie substituiertes Ammonium, Sulfonium, Sulfoxonium oder Phosphonium in Betracht. Die Erfindung umfaßt daher auch vorzugsweise diese Salze der Verbindungen der Formel (I).

Von besonderem Interesse sind Verbindungen der Formel (I) oder deren Salze bei denen

- n eine ganze Zahl von 0 bis 4, vorzugsweise 0 bis 3,
- $R^1$ gleiche oder verschiedene Substituenten aus der Gruppe gradkettiges $C_1$-$C_3$-Alkyl, i-Propyl, $C_4$-$C_6$-Cycloalkyl und Phenyl, wobei die vorstehenden 4 Reste unsubstituiert oder durch ein oder mehrere Reste aus der Gruppe Chlor und Fluor substituiert sind,
- $R^2$ Halogen, $NO_2$, CN, $C_1$-$C_3$-Alkyl, $C_1$-$C_3$-Halogenalkyl, $C_1$-$C_2$-Alkoxy, $C_1$-$C_2$-Halogenalkoxy, $R^aSO_2$-, wobei $R^a$ die oben angegebene Bedeutung hat,
- $R^3$ H, Halogen, $C_1$-$C_2$-Alkyl, $C_1$-$C_2$-Halogenalkyl, $C_1$-$C_2$-Alkoxy, $C_1$-$C_2$-Halogenalkoxy, $C_1$-$C_2$-Alkylthio und
- $R^4$ $C_1$-$C_3$-Halogenalkyl, vorzugsweise $C_1$-$C_2$-Halogenalkyl,

bedeuten.

- n ist vorzugsweise 0 bis 3;
- $R^1$ ist vorzugsweise $C_1$-$C_3$-Alkyl, inbesondere Methyl, Ethyl oder i-Propyl, Cyclopentyl oder Phenyl;
- $R^2$ ist vorzugsweise Fluor, Chlor, $NO_2$, CN, Methyl, Trifluormethyl, Methoxy, Difluormethoxy, Trifluormethoxy oder Methylsulfonyl;
- $R^3$ ist vorzugsweise Wasserstoff, Halogen, z.B. Fluor oder Chlor, Methyl, $CF_3$, Methoxy, Difluormethoxy, Trifluormethoxy oder Methylthio, insbesondere Wasserstoff;
- $R^4$ ist beispielweise $CH_2F$, $CHF_2$, $CH_2Cl$, $CHCl_2$, $CCl_3$, $CH_2CH_2Cl$, $CH_2CH_2F$, $CH_2CHF_2$, $CH_2CF_3$, $CHF$-$CH_3$, $CHF$-$CH_2F$, $CHFCHF_2$, $CF_2$-$CH_3$, $CF_2$-$CH_2F$, $CF_2CHF_2$, $CF_2$-$CF_3$, $CF_2CHFCl$, $CHFCHFCl$, $CH_2CCl_3$, $CH_2CHCl_2$, $CHFCHCl_2$, $CF_2CHCl_2$, $CHFCH_2Cl$ oder $CF_2CH_2Cl$,
- $R^4$ ist besonders $CH_2F$, $CHF_2$, $CH_2CF_3$, $CF_2CHF_2$, $CF_2CHFCl$ oder $CH_2CH_2Cl$, ganz besonders $CHF_2$.

Bevorzugt sind auch erfindungsgemäße Verbindungen der Formel (I) oder deren Salze, die eine Kombination der obengenannten bevorzugten Merkmale aufweisen.

Weiterer Gegenstand der vorliegenden Erfindung sind Verfahren zur Herstellung der Verbindungen der genannten Formel (I) oder deren Salzen dadurch gekennzeichnet, daß man

a) ein Cyclohexandion der Formel (II)

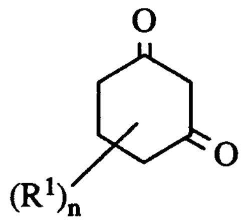

(II)

mit einem Säurechlorid der Formel (III)

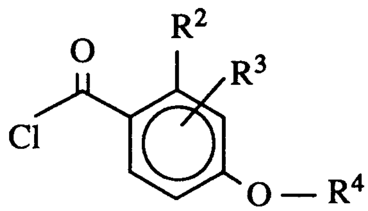

(III)

zu einem Enolether der Formel (IV)

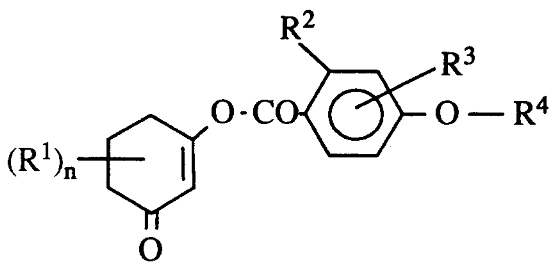

(IV)

umsetzt und diesen in Gegenwart einer Cyanid-Quelle (z.B. Acetoncyanhydrin) oder einer Lewis-Säure (z.B. $AlCl_3$) zu einer Verbindung der genannten Formel (I) umlagert
oder
b) ein Cyclohexandion der genannten Formel (II) mit einem Benzoylcyanid der Formel (V)

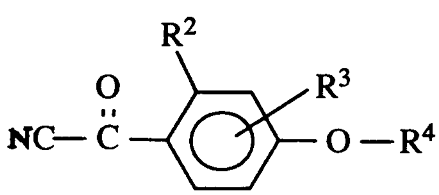

(V)

umsetzt, wobei in den Formeln (II) bis (V) $R^1$, $R^2$, $R^3$, $R^4$ und n die in Formel (I) genannte Bedeutung haben.

Die Säurechloride der Formel (III) können nach oder analog literaturbekannten Verfahren aus den Carbonsäuren der Formel (VI)

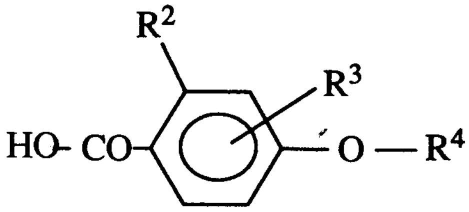

(VI)

hergestellt werden (vgl. z.B. Fieser u. Fieser, Reagents for Organic Synthesis, Vol. I, S. 1158-1159 (1967), ebenda S. 767-769).

Die Umsetzung der Verbindungen der Formeln (II) und (III) nach Variante a) wird in der Regel in Gegenwart eines organischen Lösungsmittels, z.B. eines protischen oder aprotischen Lösungsmittels durchgeführt. Geeignete Lösungsmittel sind beispielsweise aromatische oder aliphatische, gegebenenfalls halogenierte, Kohlenwasserstoffe, Ether, Ester, Nitrile, Formamide, Lactone und Alkohole und Gemische der genannten Lösungsmittel. Bevorzugt sind als Lösungsmittel aprotische, dipolare Lösungsmittel wie Ether, Acetonitril und Dimethylformamid und Gemische der Lösungsmittel. Die Umsetzung erfolgt vorzugsweise in Gegenwart einer organischen oder anorganischen Base, vorzugsweise einer sterisch gehinderten organischen Base aus der Gruppe der substituierten Amine, wie Triethylamin oder 1,8-Diazabicylo[5,4,0]undec-7-en.

Die Reaktionstemperatur ist in der Regel bei -20 ° C bis zum Siedepunkt des Lösungsmittels, vorzugsweise bei 15 ° C bis 40 ° C. Die Umlagerung des Zwischenprodukts der Formel (IV) aus der Reaktion von (II) und (III) erfolgt vorzugsweise ohne vorherige Isolierung des Zwischenprodukts durch Zugabe einer Cyanid-Quelle oder einer Lewis-Säure zum erhaltenen Reaktionsgemisch aus der Umsetzung von (II) und (III) bei -20 ° C bis zum Siedepunkt des Lösungsmittels, vorzugsweise bei 15 bis 30 ° C. Als Cyanid-Quelle eignet sich beispielsweise Acetoncyanhydrin, als Lewis-Säure $AlCl_3$.

Die Isolierung des Produkts nach der Umlagerung kann nach üblichen Methoden erfolgen, beispielsweise durch Extraktion des wäßrig-sauer gestellten Reaktionsgemisches mit einem organischen Lösungsmittel und Kristallisation.

Die Umsetzung der Verbindung der Formeln (II) und (V) nach Variante b) werden in der Regel ebenfalls in einem organischen Lösungsmittel durchgeführt, wobei die für das Verfahren nach Variante a) genannten in Frage kommen. Reaktionstemperaturen von 0 ° C bis zum Siedepunkt des Lösungsmittels sind bevorzugt, insbesondere 15 ° C bis 80 ° C.

Die Benzoylcyanide (V) können nach literaturbekannten Verfahren aus den Carbonsäuren (VI) hergestellt werden (vgl. z.B. Oakwood, Weisgerber, Organic Synthesis, Col.Vol. III, S. 112 (1955)).

Zur Herstellung substituierter Benzoesäuren ist in der chemischen Fachliteratur eine Vielzahl von Methoden beschrieben. Diese kommen auch zur Herstellung der Säuren der Formel (VI) in Betracht. Eine häufig angewandte Methode ist die Oxidation von Toluolen der Formel (VII) (z.B. mit Kaliumpermanganat oder Salpetersäure):

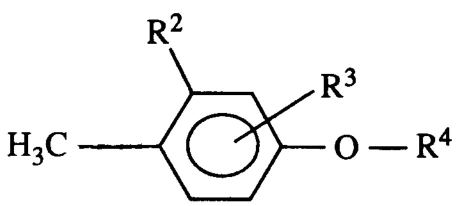

(VII)

Die Carbonsäuren der Formel (VI), ihre Salze mit anorganischen und organischen Basen und ihre reaktiven Derivate der Formel (III) und (V) sind neue Verbindungen. Gegenstand der vorliegenden Erfindung sind daher auch Verbindungen der Formel (VIII),

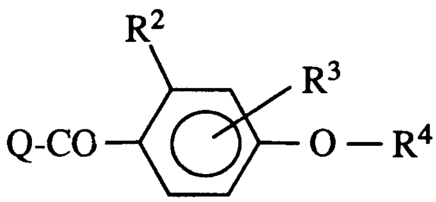

(VIII)

worin Q Cl, CN, OH oder $O^{-}Me^{+}$, worin $Me^{+}$ ein anorganisches oder organisches Kation ist, bedeutet, und $R^2$, $R^3$ und $R^4$ die obengenannte Bedeutung haben.

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflautverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z.B.. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vordem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltstoffen und zur Ernteerleichterung wie z.B. durch Auslösen von Desikkation und Wuchstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) oder deren Salze enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV- Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Valkenburg, "Pesticide Formulations", Marcel Dekker N.Y., 1973; K. Martens, "Spray Drying Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v. Olphen, "Introduction to Clay Colloid Chemistry"; 2nd Ed., J. Wiley & Sons, N.Y.; Marsden, "Solvents Guide", 2nd Ed., Interscience, N.Y. 1950; McCutcheon's "Detergents and Emulsifiers Annual", MG Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungsmittel oder Inertstoff noch Netzmittel, z.B. polyoxyethylierte Alkylphenole, polyoxethylierte Fettalkohole und Fettamine, Fettalkoholpolyglykolethersulfate, Alkansulfonate oder Alkylbenzolsulfonate und Dispergiermittel, z.B. ligninsulfonsaures Natrium, 2,2'-dinaphthylmethan-6,6'-disulfonsaures Natrium, dibutylnaphthalin-sulfonsaures Natrium oder auch oleylmethyltaurinsaures Natrium enthalten.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Cadodecylbenzolsulfonat oder nichtionische Emulgatoren wie Fettsäurepolyglykolester, Alkylarylpolyglykolether, Fettalkoholpolyglykolether, Propylenoxid-Ethylenoxid - Kondensationsprodukte, Alkylpolyether, Sorbitanfettsäureester, Polyoxyethylensorbitanfettsäureester oder Polyoxethylensorbitester.

Stäubemittel erhält man durch Vermahlen des Wirkstoffes mit fein verteilten festen Stoffen, z.B. Talkum, natürlichen Tonen, wie Kaolin, Bentonit und Pyrophyllit, und Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Die agrochemischen Zubereitungen enthalten in der Regel 0,1 bis 99 Gewichtsprozent, insbesondere 0,1 bis 95 Gew.-%, Wirkstoff der Formel (I).

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 85 Gew.-% betragen. Staubförmige Formulierungen enthalten meistens 1 bis 25 Gew.-% an Wirkstoff, versprühbare Lösungen etwa 0,2 bis 20 Gew.-%. Bei Granulaten hängt der Wirkstoffgehalt zum Teil davon ab, ob die wirksame Verbindung flüssig oder fest vorliegt. In der Regel liegt der Gehalt bei den in Wasser dispergierbaren Granulaten zwischen 10 und 90 Gew.-%.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Granulate zur Boden- bzw. Streuapplikation sowie versprühbare Lösungen werden vor der Anwendung üblicherweise nicht mehr mit weiteren inerten Stoffen verdünnt.

Mit den äußeren Bedingungen wie Temperatur Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

Auch Mischungen oder Mischformulierungen mit anderen Wirkstoffen, wie z.B. Insektiziden, Akariziden, Herbiziden, Safenern, Düngemitteln, Wachstumsregulatoren oder Fungiziden sind gegebenenfalls möglich.

A. Chemische Beispiele

Beispiel A: 2-(2-Chlor-4-difluormethoxy-benzoyl)-cyclohexan-1,3-dion (Verbindung Nr. 18):

7,23 g 2-Chlor-4-difluormethoxybenzoylchlorid und 2,80 g 1,3-Cyclohexandion werden in 30 ml Acetonitril vorgelegt und 6,32 g Triethylamin so zugetropft, daß die Innentemperatur 40 °C nicht übersteigt. Anschließend rührt man 15 min bei Raumtemperatur, fügt 1,95 g Acetoncyanhydrin zu und rührt weiterhin 3 h bei Raumtemperatur. Das Reaktionsgemisch verdünnt man mit 100 ml Äthylacetat und fügt 50 ml 1N Salzsäure hinzu. Nach intensivem Schütteln werden die Phasen getrennt und die organische Phase zweimal mit je 50 ml 5%-iger Kaliumcarbonat-Lösung extrahiert. Die vereinigten wäßrigen Phasen werden mit konzentrierter Salzsäure sauer gestellt und erneut mit Dichlormethan (2 mal 50 ml) extrahiert. Nach Trocknen über Magnesiumsulfat und Abziehen des Lösungsmittels erhält man 6,9 g (87 % d. Th. ) 2-(2-Chlor-4-difluormethoxybenzoyl)-cyclohexan-1,3-dion als blaßgelbe Kristalle mit einem Schmelzpunkt 68 - 72 °C.

Beispiel B: 2-(2-Nitro-4-difluormethoxybenzoyl)-4,4,6-trimethylcyclohexan-1,3-dion (Verbindung Nr. 71)

5,03 g 2-Nitro-4-difluormethoxybenzoylchlorid und 2,57g 4,4,6-Trimethylcyclohexan-1,3-dion werden in 20 ml Acetonitril vorgelegt und 4,21 g Triethylamin so zugetropft, daß die Innentemperatur 40 °C nicht übersteigt. Anschließend rührt man 15 min bei Raumtemperatur, gibt 1,29 g Acetoncyanhydrin zu und rührt weiterhin 3 h bei Raumtemperatur. Das Reaktionsgemisch verdünnt man mit 100 ml Äthylacetat und fügt 50 ml 1N Salzsäure hinzu. Nach intensivem Schütteln werden die Phasen getrennt, die organischen Phasen über $MgS0_4$ getrocknet und das Lösungsmittel unter reduziertem Druck abgezogen. Das verbleibende Öl (7,1g) chromatographiert man an Kieselgel mit Äthylacetat/Petrolether 1:2 als Elutionsmittel. Man erhält 3,28 g (46 % d.Th.) 2-(2-Nitro-4-difluormethoxy-benzoyl)-4,4,6-trimethylcyclohexan-1,3-dion mit einem Schmelzpunkt von 108 - 110 °C.

Beispiel C: 2-Nitro-4-difluormethoxybenzoyl-chlorid

23,3 g 2-Nitro-4-difluormethoxybenzoesäure und 16 g Thionylchlorid werden mit 200 mg Dimethylformamid 7 h zum Rückfluß erhitzt. Anschließend wird das Reaktionsgemisch unter reduziertem Druck destilliert. Man erhält 23,7 g Produkt vom Siedepunkt 101 - 105 °C bei 0,4 mbar.

Beispiel D: 2-Nitro-4-difluormethoxy-benzoesäure

72,1g 2-Nitro-4-difluormethoxytoluol und 300 ml Pyridin werden zum Rückfluß erhitzt. Portionsweise wird dann eine Lösung von 336 g Kaliumpermanganat in 500 ml Wasser zugesetzt. Anschließend hält man den Ansatz noch 7 h bei 80 °C. Nach dem Erkalten gibt man 500 ml 2N Natriumlauge zu, extrahiert mit Ether und stellt die Wasserphase mit konzentrierter Salzsäure sauer. Dabei scheidet sich das Produkt als Öl ab, das nach längerem Rühren kristallisiert. Nach Filtration, Waschen mit verdünnter Salzsäure und Trocknen erhält man 53,6 g gewünschtes Produkt als weiße Kristalle (Fp. 136 - 138 °C).

Die Verbindungen aus der folgenden Tabelle 1 werden analog zu den in den Bespielen A und B beschriebenen Verfahren erhalten. Die dafür eingesetzten Benzoylchloride werden analog den Verfahren der Beispiele C und D erhalten.

Tabelle 1: Erfindungsgemäße Verbindungen der Formel (I)

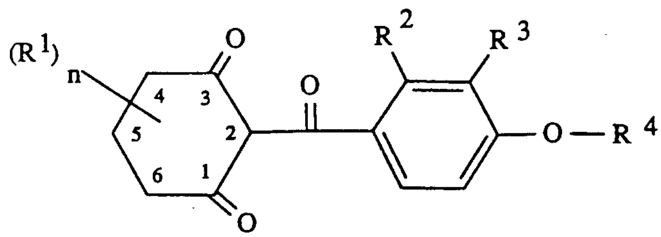

(I)

| Nr. | $R^1$ | n | $R^2$ | $R^3$ | O-$R^4$ | Fp |
|---|---|---|---|---|---|---|
| 1 | | 0 | F | H | $OCH_2F$ | |
| 2 | | 0 | F | H | $OCHF_2$ | 122-123 |
| 3 | 4-$CH_3$ | 1 | F | H | $OCHF_2$ | |
| 4 | 5-$CH_3$ | 1 | F | H | $OCHF_2$ | |
| 5 | 4,4-$(CH_3)_2$ | 2 | F | H | $OCHF_2$ | 123-124 |
| 6 | 5,5-$(CH_3)_2$ | 2 | F | H | $OCHF_2$ | |
| 7 | 4,5-$(CH_3)_2$ | 2 | F | H | $OCHF_2$ | |
| 8 | 4,6-$(CH_3)_2$ | 2 | F | H | $OCHF_2$ | |
| 9 | 4,4,5-$(CH_3)_3$ | 3 | F | H | $OCHF_2$ | |
| 10 | 4,4,6-$(CH_3)_3$ | 3 | F | H | $OCHF_2$ | |
| 11 | 4,5,5-$(CH_3)_3$ | 3 | F | H | $OCHF_2$ | |
| 12 | 4,5,6-$(CH_3)_3$ | 3 | F | H | $OCHF_2$ | |
| 13 | | 0 | F | Cl | $OCHF_2$ | |
| 14 | | 0 | F | $CH_3$ | $OCHF_2$ | |
| 15 | | 0 | F | $CF_3$ | $OCHF_2$ | |
| 16 | | 0 | F | $OCH_3$ | $OCHF_2$ | |
| 17 | | 0 | Cl | H | $OCH_2F$ | |
| 18 | | 0 | Cl | H | $OCHF_2$ | 68 - 72 |
| 19 | | 0 | CL | H | $OCH_2CF_3$ | |
| 20 | | 0 | Cl | H | $OCF_2CHF_2$ | |
| 21 | | 0 | Cl | H | $OCF_2CHFCl$ | |
| 22 | | 0 | Cl | H | $OCH_2CH_2Cl$ | |
| 23 | 4-$CH_3$ | 1 | Cl | H | $OCHF_2$ | Oel |
| 24 | 4-$CH_3$ | 1 | Cl | H | $OCH_2F$ | |
| 25 | 5-$CH_3$ | 1 | Cl | H | $OCHF_2$ | |
| 26 | 5-$CH_3$ | 1 | Cl | H | $OCH_2F$ | |
| 27 | 4,4-$(CH_3)_2$ | 2 | Cl | H | $OCHF_2$ | Oel |

Fortsetzung Tabelle 1

| Nr. | $R^1$ | n | $R^2$ | $R^3$ | $O-R^4$ | Fp |
|---|---|---|---|---|---|---|
| 28 | 4,4-$(CH_3)_2$ | 2 | Cl | H | $OCH_2F$ | |
| 29 | 5,5-$(CH_3)_2$ | 2 | Cl | H | $OCHF_2$ | Oel |
| 30 | 5,5-$(CH_3)_2$ | 2 | Cl | H | $OCH_2F$ | |
| 31 | 4,5-$(CH_3)_2$ | 2 | Cl | H | $OCHF_2$ | Oel |
| 32 | 4,6-$(CH_3)_2$ | 2 | Cl | H | $OCHF_2$ | 114 |
| 33 | 4,4,5-$(CH_3)_3$ | 3 | Cl | H | $OCHF_2$ | Oel |
| 34 | 4,4,5-$(CH_3)_3$ | 3 | Cl | H | $OCH_2F$ | |
| 35 | 4,4,6-$(CH_3)_3$ | 3 | Cl | H | $OCHF_2$ | 108 - 110 |
| 36 | 4,4,6-$(CH_3)_3$ | 3 | Cl | H | $OCH_2F$ | |
| 37 | 4,5,5-$(CH_3)_3$ | 3 | Cl | H | $OCHF_2$ | |
| 38 | 4,5,6-$(CH_3)_3$ | 3 | Cl | H | $OCHF_2$ | |
| 39 | 4-$CH_2CH_3$ | 1 | Cl | H | $OCHF_2$ | 125 |
| 40 | 4-$CH(CH_3)_2$ | 1 | Cl | H | $OCHF_2$ | Oel |
| 41 | 4-Cyclopentyl | 1 | Cl | H | $OCHF_2$ | |
| 42 | 4-Ph | 1 | Cl | H | $OCHF_2$ | Oel |
| 43 | 5-$CH(CH_3)_2$ | 1 | Cl | H | $OCHF_2$ | |
| 44 | | 0 | Cl | F | $OCHF_2$ | |
| 45 | | 0 | Cl | Cl | $OCH_2F$ | |
| 46 | | 0 | Cl | Cl | $OCHF_2$ | |
| 47 | | 0 | Cl | $CH_3$ | $OCHF_2$ | |
| 48 | 4,4-$(CH_3)_2$ | 2 | Cl | $CH_3$ | $OCHF_2$ | |
| 49 | | 0 | Cl | $CF_3$ | $OCHF_2$ | |
| 50 | | 0 | Cl | $OCH_3$ | $OCHF_2$ | |
| 51 | | 0 | Cl | $OCHF_2$ | $OCHF_2$ | |
| 52 | | 0 | Cl | $SCH_3$ | $OCHF_2$ | |
| 53 | | 0 | $NO_2$ | H | $OCH_2F$ | |
| 54 | | 0 | $NO_2$ | H | $OCHF_2$ | Oel |
| 55 | | 0 | $NO_2$ | H | $OCH_2CF_3$ | |
| 56 | | 0 | $NO_2$ | H | $OCF_2CHF_2$ | |
| 57 | | 0 | $NO_2$ | H | $OCF_2CHFCl$ | 130(Zers.) |
| 58 | | 0 | $NO_2$ | H | $OCH_2CH_2Cl$ | |
| 59 | 4-$CH_3$ | 1 | $NO_2$ | H | $OCHF_2$ | Oel |
| 60 | 4-$CH_3$ | 1 | $NO_2$ | H | $OCH_2F$ | |
| 61 | 5-$CH_3$ | 1 | $NO_2$ | H | $OCHF_2$ | 120-122 |
| 62 | 5-$CH_3$ | 1 | $NO_2$ | H | $OCH_2F$ | |
| 63 | 4,4-$(CH_3)_2$ | 2 | $NO_2$ | H | $OCHF_2$ | Oel |

Fortsetzung Tabelle 1

| Nr. | $R^1$ | n | $R^2$ | $R^3$ | $O\text{-}R^4$ | Fp |
|---|---|---|---|---|---|---|
| 64 | 4,4-$(CH_3)_2$ | 2 | $NO_2$ | H | $OCH_2F$ | |
| 65 | 5,5-$(CH_3)_2$ | 2 | $NO_2$ | H | $OCHF_2$ | Oel |
| 66 | 5,5-$(CH_3)_2$ | 2 | $NO_2$ | H | $OCH_2F$ | |
| 67 | 4,5-$(CH_3)_2$ | 2 | $NO_2$ | H | $OCHF_2$ | Oel |
| 68 | 4,6-$(CH_3)_2$ | 2 | $NO_2$ | H | $OCHF_2$ | |
| 69 | 4,4,5-$(CH_3)_3$ | 3 | $NO_2$ | H | $OCHF_2$ | Oel |
| 70 | 4,4,5-$(CH_3)_3$ | 3 | $NO_2$ | H | $OCH_2F$ | |
| 71 | 4,4,6-$(CH_3)_3$ | 3 | $NO_2$ | H | $OCHF_2$ | Oel |
| 72 | 4,4,6-$(CH_3)_3$ | 3 | $NO_2$ | H | $OCH_2F$ | |
| 73 | 4,5,5-$(CH_3)_3$ | 3 | $NO_2$ | H | $OCHF_2$ | |
| 74 | 4,5,6-$(CH_3)_3$ | 3 | $NO_2$ | H | $OCHF_2$ | |
| 75 | 4-$CH_2CH_3$ | 1 | $NO_2$ | H | $OCHF_2$ | |
| 76 | 4-$CH(CH_3)_2$ | 1 | $NO_2$ | H | $OCHF_2$ | Oel |
| 77 | 4-Cyclopentyl | 1 | $NO_2$ | H | $OCHF_2$ | |
| 78 | 4-Ph | 1 | $NO_2$ | H | $OCHF_2$ | 170 |
| 79 | 5-$CH(CH_3)_2$ | 1 | $NO_2$ | H | $OCHF_2$ | |
| 80 | | 0 | $NO_2$ | F | $OCHF_2$ | |
| 81 | | 0 | $NO_2$ | Cl | $OCH_2F$ | |
| 82 | | 0 | $NO_2$ | Cl | $OCHF_2$ | |
| 83 | | 0 | $NO_2$ | $CH_3$ | $OCHF_2$ | |
| 84 | 4,4-$(CH_3)_2$ | 2 | $NO_2$ | $CH_3$ | $OCHF_2$ | |
| 85 | | 0 | $NO_2$ | $CF_3$ | $OCHF_2$ | |
| 86 | | 0 | $NO_2$ | $OCH_3$ | $OCHF_2$ | |
| 87 | | 0 | $NO_2$ | $OCHF_2$ | $OCHF_2$ | |
| 88 | | 0 | $NO_2$ | $SCH_3$ | $OCHF_2$ | |
| 89 | | 0 | CN | H | $OCH_2F$ | |
| 90 | | 0 | CN | H | $OCHF_2$ | |
| 91 | 4-$CH_3$ | 1 | CN | H | $OCHF_2$ | |
| 92 | 5-$CH_3$ | 1 | CN | H | $OCHF_2$ | |
| 93 | 4,4-$(CH_3)_2$ | 2 | CN | H | $OCHF_2$ | |
| 94 | 5,5-$(CH_3)_2$ | 2 | CN | H | $OCHF_2$ | |
| 95 | 4,4,5-$(CH_3)_3$ | 3 | CN | H | $OCHF_2$ | |
| 96 | 4,4,6-$(CH_3)_3$ | 3 | CN | H | $OCHF_2$ | |
| 97 | | 0 | CN | Cl | $OCHF_2$ | |
| 98 | | 0 | CN | $CH_3$ | $OCHF_2$ | |
| 99 | | 0 | CN | $OCH_3$ | $OCHF_2$ | |

Fortsetzung Tabelle 1

| Nr. | $R^1$ | n | $R^2$ | $R^3$ | $O-R^4$ | Fp |
|---|---|---|---|---|---|---|
| 100 | | 0 | $CH_3$ | H | $OCH_2F$ | |
| 101 | | 0 | $CH_3$ | H | $OCHF_2$ | |
| 102 | $4-CH_3$ | 1 | $CH_3$ | H | $OCHF_2$ | |
| 103 | $5-CH_3$ | 1 | $CH_3$ | H | $OCHF_2$ | |
| 104 | $4,4-(CH_3)_2$ | 2 | $CH_3$ | H | $OCHF_2$ | |
| 105 | $5,5-(CH_3)_2$ | 2 | $CH_3$ | H | $OCHF_2$ | |
| 106 | $4,4,5-(CH_3)_3$ | 3 | $CH_3$ | H | $OCHF_2$ | |
| 107 | $4,4,6-(CH_3)_3$ | 3 | $CH_3$ | H | $OCHF_2$ | |
| 108 | | 0 | $CH_3$ | Cl | $OCHF_2$ | |
| 109 | | 0 | $CH_3$ | $OCH_3$ | $OCHF_2$ | |
| 110 | | 0 | $CF_3$ | H | $OCH_2F$ | |
| 111 | | 0 | $CF_3$ | H | $OCHF_2$ | |
| 112 | $4-CH_3$ | 1 | $CF_3$ | H | $OCHF_2$ | |
| 113 | $5-CH_3$ | 1 | $CF_3$ | H | $OCHF_2$ | |
| 114 | $4,4-(CH_3)_2$ | 2 | $CF_3$ | H | $OCHF_2$ | |
| 115 | $5,5-(CH_3)_2$ | 2 | $CF_3$ | H | $OCHF_2$ | |
| 116 | $4,4,5-(CH_3)_3$ | 3 | $CF_3$ | H | $OCHF_2$ | |
| 117 | $4,4,6-(CH_3)_3$ | 3 | $CF_3$ | H | $OCHF_2$ | |
| 118 | | 0 | $CF_3$ | Cl | $OCHF_2$ | |
| 119 | | 0 | $CF_3$ | $OCH_3$ | $OCHF_2$ | |
| 120 | | 0 | $OCH_3$ | H | $OCH_2F$ | |
| 121 | | 0 | $OCH_3$ | H | $OCHF_2$ | |
| 122 | $4-CH_3$ | 1 | $OCH_3$ | H | $OCHF_2$ | |
| 123 | $4,4-(CH_3)_2$ | 2 | $OCH_3$ | H | $OCHF_2$ | |
| 124 | $5,5-(CH_3)_2$ | 2 | $OCH_3$ | H | $OCHF_2$ | |
| 125 | $4,4,5-(CH_3)_3$ | 3 | $OCH_3$ | H | $OCHF_2$ | |
| 126 | $4,4,6-(CH_3)_3$ | 3 | $OCH_3$ | H | $OCHF_2$ | |
| 127 | | 0 | $OCHF_2$ | H | $OCHF_2$ | |
| 128 | $4-CH_3$ | 1 | $OCHF_2$ | H | $OCHF_2$ | |
| 129 | $4,4-(CH_3)_2$ | 2 | $OCHF_2$ | H | $OCHF_2$ | |
| 130 | $5,5-(CH_3)_2$ | 2 | $OCHF_2$ | H | $OCHF_2$ | |
| 131 | $4,4,5-(CH_3)_3$ | 3 | $OCHF_2$ | H | $OCHF_2$ | |
| 132 | $4,4,6-(CH_3)_3$ | 3 | $OCHF_2$ | H | $OCHF_2$ | |
| 133 | | 0 | $SCH_3$ | H | $OCHF_2$ | |
| 134 | $4,4-(CH_3)_2$ | 2 | $SCH_3$ | H | $OCHF_2$ | |
| 135 | $4,4,6-(CH_3)_3$ | 3 | $SCH_3$ | H | $OCHF_2$ | |

Fortsetzung Tabelle 1

| Nr. | $R^1$ | n | $R^2$ | $R^3$ | $O\text{-}R^4$ | Fp |
|---|---|---|---|---|---|---|
| 136 | | 0 | $SO_2CH_3$ | H | $OCH_2F$ | |
| 137 | | 0 | $SO_2CH_3$ | H | $OCHF_2$ | |
| 138 | $4\text{-}CH_3$ | 1 | $SO_2CH_3$ | H | $OCHF_2$ | |
| 139 | $5\text{-}CH_3$ | 1 | $SO_2CH_3$ | H | $OCHF_2$ | |
| 140 | $4,4\text{-}(CH_3)_2$ | 2 | $SO_2CH_3$ | H | $OCHF_2$ | |
| 141 | $5,5\text{-}(CH_3)_2$ | 2 | $SO_2CH_3$ | H | $OCHF_2$ | |
| 142 | $4,4,5\text{-}(CH_3)_3$ | 3 | $SO_2CH_3$ | H | $OCHF_2$ | |
| 143 | $4,4,6\text{-}(CH_3)_3$ | 3 | $SO_2CH_3$ | H | $OCHF_2$ | |
| 144 | | 0 | $SO_2CH_3$ | Cl | $OCHF_2$ | |
| 145 | | 0 | $SO_2CH_3$ | $OCH_3$ | $OCHF_2$ | |

B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.

b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen (8 EO) und 71 Gew.-Teilen paraffinischem Mineralöl (Siedebereich z.B. ca. 255 bis über 277 °C) mischt und in einer Reibkugelmühle auf eine Feinheit von unter 5 Mikron vermahlt.

d) Ein emulgierbares Konzentrat wird erhalten aus 15 Gew.Teilen einer Verbindung der Formel (I), 75 Gew.-Teilen Cyclohexan als Lösungsmittel und 10 Gew.-Teilen oxethyliertes Nonylphenol als Emulgator.

e) Ein in Wasser dispergierbares Granulat wird erhalten, indem man

| | | |
|---|---|---|
| 75 Gewichtsteile einer Verbindung der Formel (I), | | |
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftmühle mahlt und das Pulver in einem Wirbelbett durch Aufsprühen von Wasser als Granulierflüssigkeit granuliert.

f) Ein in Wasser dispergierbares Granulat wird auch erhalten, indem man

| | | |
|---|---|---|
| 25 | Gewichtsteile einer | Verbindung der Formel (I), |
| 5 | " | 2,2'-dinaphthylmethan-6,6'-disulfonsaures Natrium, |
| 2 | " | oleylmethyltaurinsaures Natrium, |
| 1 | Gewichtsanteil | Polyvinylalkohol, |
| 17 | Gewichtsanteile | Calciumcarbonat und |
| 50 | " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

g) Ein Extruder-Granulat erhält man, indem man 20 Gewichtsteile Wirkstoff, 3 Gewichtsteile ligninsulfonsaures Natrium, 1 Gewichtsteil Carboxymethylcellulose und 76 Gewichtsteile Kaolin vermischt, vermahlt und mit Wasser anfeuchtet. Dieses Gemisch wird extrudiert und anschließend in Luftstrom getrocknet.

C. Biologische Beispiele

Die Schädigung der Schadpflanzen bzw. die Kulturpflanzenverträglichkeit wurde gemäß einem Schlüssel bonitiert, in dem die Wirksamkeit durch Wertzahlen von 0 bis 5 ausgedrückt ist. Dabei bedeutet

0 = ohne Wirkung
1 = 0 bis 20 % Wirkung bzw. Schaden
2 = 20 bis 40 % Wirkung bzw. Schaden
3 = 40 bis 60 % Wirkung bzw. Schaden
4 = 60 bis 80 % Wirkung bzw. Schaden
5 = 80 bis 100 % Wirkung bzw. Schaden

1. Unkrautwirkung im Vorauflauf

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 bis 4 Wochen im Vergleich zu unbehandelten Kontrollen. Wie die Boniturwerte zeigen, weisen die erfindungsgemäßen Verbindungen eine gute herbizide Vorauflaufwirksamkeit gegen ein breites Spektrum von Ungräsern und Unkräutern auf (vgl. Tabelle 2).

Tabelle 2

| Vorauflaufwirkung erfindungsgemäßer Verbindungen | | | | | | | |
|---|---|---|---|---|---|---|---|
| Bsp. Nr. | Dosis (kg a.i./ha) | STME | CRSE | SIAL | LOMU | ECCR | AVSA |
| 18 | 1,25 | 5 | 5 | 5 | 2 | 5 | 2 |
| 27 | 1,25 | 5 | 5 | 5 | 5 | 5 | 5 |
| 29 | 1,25 | 5 | 5 | 5 | 3 | 5 | 2 |
| 33 | 1,25 | 5 | 5 | 5 | 4 | 5 | 2 |
| 35 | 1,25 | 5 | 5 | 5 | 5 | 5 | 3 |
| 42 | 1,25 | 4 | 4 | 1 | 2 | 2 | 0 |
| 54 | 1,25 | 5 | 5 | 5 | 5 | 5 | 5 |
| 63 | 1,25 | 5 | 5 | 5 | 5 | 5 | 5 |
| 65 | 1,25 | 5 | 5 | 5 | 5 | 5 | 3 |
| 69 | 1,25 | 5 | 5 | 5 | 5 | 5 | 3 |
| 71 | 1,25 | 5 | 5 | 5 | 5 | 5 | 4 |
| 76 | 1,25 | 5 | 5 | 5 | 5 | 5 | 3 |
| 78 | 1,25 | 5 | 5 | 5 | 4 | 5 | 3 |

Abkürzungen:
STME = Stellaria media
CRSE = Chrysanthemum segetum
SIAL = Sinapis alba
LOMU = Lolium multiflorum
ECCR = Echinochloa crus-galli
AVSA = Avena sativa
a.i. = active ingredient (= bezogen auf reinen Wirkstoff)

Eine vergleichbar gute Vorauflaufwirkung wie bei den in Tabelle 2 aufgeführten Beispielen haben beispielsweise auch die Verbindungen der Beispiele 23, 40, 31, 57, 59, 61 und 67 aus Tabelle 1 gegen Schadpflanzen wie Avena fatua, Alopecurus myosuroides, Setaria pumila, Poa annua, Stellaria media, Pharbitis purpura, Lolium multiflorum, Echinochloa crus-galli, Cyperus esculentus, Sinapis alba, Galium aparine, Chrysanthemum segetum, Matricaria inodora und Amaranthus retroflexus.

2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Spektrum wirtschaftlich wichtiger Ungräser und Unkräuter auf (vgl. Tabelle 3).

Tabelle 3

| Nachauflaufwirkung der erfindungsgemäßen Verbindungen | | | | | | | |
|---|---|---|---|---|---|---|---|
| Bsp. Nr. | Dosis (kg a.i./ha) | STME | CRSE | SIAL | LOMU | ECCR | AVSA |
| 18 | 1,25 | 5 | 2 | 5 | 1 | 4 | 1 |
| 27 | 1,25 | 4 | 4 | 5 | 4 | 4 | 3 |
| 29 | 1,25 | 4 | 3 | 5 | 1 | 4 | 0 |
| 33 | 1,25 | 5 | 4 | 5 | 2 | 3 | 3 |
| 35 | 1,25 | 5 | 3 | 4 | 4 | 5 | 3 |
| 42 | 1,25 | 3 | 3 | 3 | 0 | 1 | 0 |
| 54 | 1,25 | 5 | 5 | 5 | 3 | 5 | 1 |
| 63 | 1,25 | 5 | 4 | 5 | 5 | 5 | 2 |
| 65 | 1,25 | 5 | 5 | 5 | 3 | 5 | 2 |
| 69 | 1,25 | 5 | 4 | 5 | 4 | 5 | 3 |
| 71 | 1,25 | 5 | 3 | 5 | 5 | 4 | 4 |
| 76 | 1,25 | 5 | 5 | 5 | 5 | 5 | 2 |
| 78 | 1,25 | 4 | 4 | 5 | 1 | 4 | 0 |
| Abkürzungen: siehe Tabelle 2 | | | | | | | |

Eine vergleichbar gute Nachauflaufwirkung wie bei den in Tabelle 3 aufgeführten Beispielen haben beispielsweise auch die Verbindungen der Beispiele 23, 40, 31, 57, 59, 61 und 67 aus Tabelle 1 gegen Schadpflanzen wie Avena fatua, Alopecurus myosuroides, Setaria pumila, Poa annua, Stellaria media, Pharbitis purpura, Lolium multiflorum, Echinochloa crus-galli, Cyperus esculentus, Sinapis alba, Galium aparine, Chrysanthemum segetum, Matricaria inodora und Amaranthus retroflexus.

3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt.

Ein Teil der Töpfe wurde sofort wie unter 1. beschrieben behandelt, die übrigen im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben, besprüht. Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z.B. Gerste, Weizen, Roggen, Sorghum-Hirsen, Mais oder Reis. Die Verbindungen der Formel (I) weisen somit eine hohe Selektivität bei Anwendung zur Bekämpfung von unerwünschtem Pflanzenwuchs in landwirtschaftlichen Kulturen auf.

## Patentansprüche

**1.** Verbindungen der Formel (I) oder deren Salze,

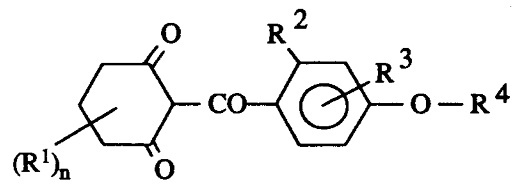 (I)

worin

n eine ganze Zahl von 0 bis 6,

$R^1$ gleiche oder verschiedene Substituenten aus der Gruppe gradkettiges $C_1$-$C_4$-Alkyl, verzweigtes $C_3$-$C_4$-Alkyl, $C_3$-$C_6$-Cycloalkyl und Phenyl; wobei die genannten Reste unsubstituiert oder durch ein oder mehrere Halogen-Atome substituiert sind,

$R^2$ Halogen, $NO_2$, CN, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Thioalkyl, $R^aSO_2$-, $R^aSO_2$-O-, $R^aSO_2NR^b$-, wobei $R^a$ und $R^b$ unabhängig voneinander $C_1$-$C_2$-Alkyl oder $C_1$-$C_2$-Halogenalkyl bedeuten,

$R^3$ Wasserstoff, Halogen, $C_1$-$C_3$-Alkyl, $C_1$-$C_3$-Halogenalkyl, $C_1$-$C_3$-Alkoxy, $C_1$-$C_3$-Halogenalkoxy oder $C_1$-$C_3$-Alkylthio und

$R^4$ $C_1$-$C_3$-Halogenalkyl bedeuten.

**2.** Verbindungen der Formel (I) oder deren Salze nach Anspruch 1, dadurch gekennzeichnet, daß

n eine ganze Zahl von 0 bis 4,

$R^1$ gleiche oder verschiedene Substituenten aus der Gruppe gradkettiges $C_1$-$C_3$-Alkyl, i-Propyl, $C_4$-$C_6$-Cycloalkyl und Phenyl, wobei die vorstehenden 4 Reste unsubstituiert oder durch ein oder mehrere Reste aus der Gruppe Chlor und Fluor substituiert sind,

$R^2$ Halogen, $NO_2$, CN, $C_1$-$C_3$-Alkyl, $C_1$-$C_3$-Halogenalkyl, $C_1$-$C_2$-Alkoxy, $C_1$-$C_2$-Halogenalkoxy, $R^aSO_2$-, wobei $R^a$ die oben angegebene Bedeutung hat,

$R^3$ H, Halogen, $C_1$-$C_2$-Alkyl, $C_1$-$C_2$-Halogenalkyl, $C_1$-$C_2$-Alkoxy, $C_1$-$C_2$-Halogenalkoxy, $C_1$-$C_2$-Alkylthio und

$R^4$ $C_1$-$C_3$-Halogenalkyl,

bedeuten.

**3.** Verbindungen der Formel (I) oder deren Salze nach Anspruch 1 oder 2, dadurch gekennzeichnet, daß

n 0 bis 3;

$R^1$ $C_1$-$C_3$-Alkyl, Cyclopentyl oder Phenyl;

$R^2$ Fluor, Chlor, $NO_2$, CN, Methyl, Trifluormethyl, Methoxy, Difluormethoxy, Trifluormethoxy oder Methylsulfonyl;

$R^3$ Wasserstoff, Halogen, Methyl, $CF_3$, Methoxy, Difluormethoxy, Trifluormethoxy oder Methylthio, und

$R^4$ $CH_2F$, $CHF_2$, $CH_2Cl$, $CHCl_2$, $CCl_3$, $CH_2CH_2Cl$, $CH_2CH_2F$, $CH_2CHF_2$, $CH_2CF_3$, $CHFCH_3$, $CHFCH_2F$, $CHFCHF_2$, $CF_2CH_3$, $CF_2CH_2F$, $CF_2CHF_2$, $CF_2CF_3$, $CF_2CHFCl$, $CHFCHFCl$, $CH_2CCl_3$, $CH_2CHCl_2$, $CHFCHCl_2$, $CF_2CHCl_2$, $CHFCH_2Cl$ oder $CF_2CH_2Cl$ bedeuten.

**4.** Verfahren zur Herstellung von Verbindungen der Formel (I) nach Anspruch 1 oder deren Salzen, dadurch gekennzeichnet, daß man

a) ein Cyclohexandion der Formel (II)

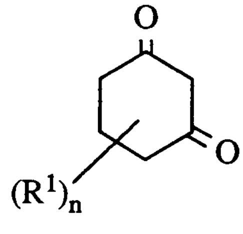 (II)

mit einem Säurechlorid der Formel (III)

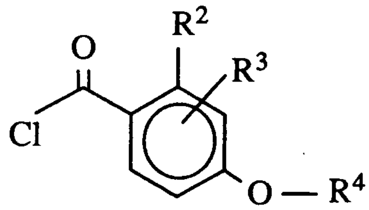

(III)

zu einem Enolether der Formel (IV)

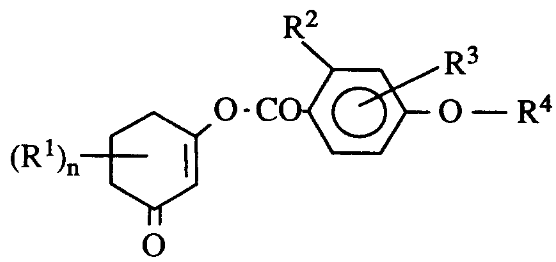

(IV)

umsetzt und diesen in Gegenwart einer Cyanid-Quelle oder einer Lewis-Säure zu einer Verbindung der genannten Formel (I) umlagert

oder
b) ein Cyclohexandion der genannten Formel (II) mit einem Benzoylcyanid der Formel (V)

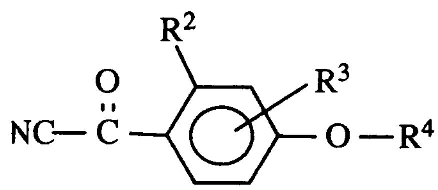

(V)

umsetzt, wobei in den Formeln (II) bis (V) $R^1$, $R^2$, $R^3$, $R^4$ und n die in Formel (I) genannte Bedeutung haben.

**5.** Herbizide oder pflanzenwachstumsregulatorische Mittel, dadurch gekennzeichnet, daß sie eine Verbindung der Formel (I) oder deren Salze nach einem oder mehreren der Ansprüche 1 bis 3 und übliche Formulierungshilfsmittel enthalten.

**6.** Verwendung von Verbindungen der Formel (I) oder deren Salze, nach einem oder mehreren der Ansprüche 1 bis 3 als Herbizide oder Pflanzenwachstumsregulatoren.

**7.** Verfahren zur selektiven Bekämpfung von Schadpflanzen, dadurch gekennzeichnet, daß man eine wirksame Menge einer der nach einem oder mehreren der Ansprüche 1 bis 3 definierten Verbindungen der Formel II oder von deren Salzen auf die Pflanzen, Pflanzensamen oder die Anbaufläche appliziert.

**8.** Verfahren zur Wachstumsregulierung von Pflanzen, dadurch gekennzeichnet, daß man eine wirksame Menge einer der nach einem oder mehreren der Ansprüche 1 bis 3 definierten Verbindungen der Formel II oder von deren Salzen auf die Pflanzen, Pflanzensamen oder die Anbaufläche appliziert.

**9.** Verbindungen der Formel (VIII)

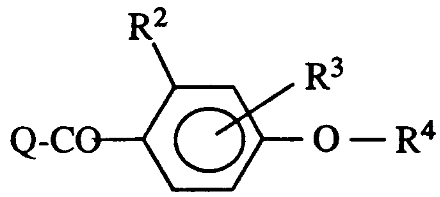 (VIII)

worin Q Cl, CN, OH oder $O^{-}Me^{+}$, worin $Me^{+}$ ein anorganisches oder organisches Kation ist, bedeutet, und $R^2$, $R^3$ und $R^4$ wie in Formel (I) nach einem oder mehreren der Ansprüche 1 bis 3 definiert sind.

**10.** Verfahren zur Herstellung der Verbindungen der Formel VIII nach Anspruch 9, dadurch gekennzeichnet, daß man entsprechende Toluole der Formel VII,

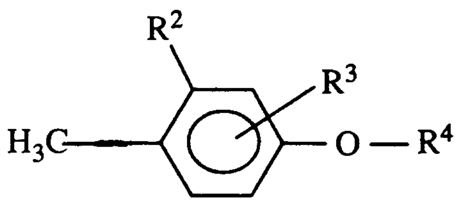 (VII)

mit Kaliumpermanganat oder Salpetersäure zu Verbindungen der Formel VIII, worin Q = COOH bedeutet, oxidiert und, im Fall Q = Cl, halogeniert oder, im Falle Q = CN, in das Benzoylcyanid umwandelt.

**Patentansprüche für folgenden Vertragsstaat : ES**

**1.** Verwendung von Verbindungen der Formel (I) oder deren Salze,

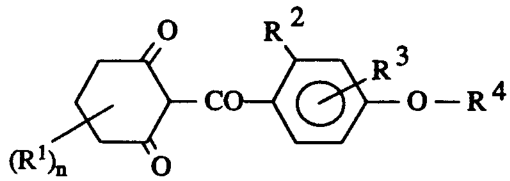 (I)

worin

- n eine ganze Zahl von 0 bis 6,
- $R^1$ gleiche oder verschiedene Substituenten aus der Gruppe gradkettiges $C_1$-$C_4$-Alkyl, verzweigtes $C_3$-$C_4$-Alkyl, $C_3$-$C_6$-Cycloalkyl und Phenyl; wobei die genannten Reste unsubstituiert oder durch ein oder mehrere Halogen-Atome substituiert sind,
- $R^2$ Halogen, $NO_2$, CN, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Thioalkyl, $R^aSO_2$-, $R^aSO_2$-O-, $R^aSO_2NR^b$-, wobei $R^a$ und $R^b$ unabhängig voneinander $C_1$-$C_2$-Alkyl oder $C_1$-$C_2$-Halogenalkyl bedeuten,
- $R^3$ Wasserstoff, Halogen, $C_1$-$C_3$-Alkyl, $C_1$-$C_3$-Halogenalkyl, $C_1$-$C_3$-Alkoxy, $C_1$-$C_3$-Halogenalkoxy oder $C_1$-$C_3$-Alkylthio und
- $R^4$ $C_1$-$C_3$-Halogenalkyl bedeuten, als Herbizide

**2.** Verwendung nach Anspruch 1, dadurch gekennzeichnet, daß

- n eine ganze Zahl von 0 bis 4,
- $R^1$ gleiche oder verschiedene Substituenten aus der Gruppe gradkettiges $C_1$-$C_3$-Alkyl, i-Propyl, $C_4$-$C_6$-Cycloalkyl und Phenyl, wobei die vorstehenden 4 Reste unsubstituiert oder durch ein oder mehrere Reste aus der Gruppe Chlor und Fluor substituiert sind,
- $R^2$ Halogen, $NO_2$, CN, $C_1$-$C_3$-Alkyl, $C_1$-$C_3$-Halogenalkyl, $C_1$-$C_2$-Alkoxy, $C_1$-$C_2$-Halogenalkoxy,

$R^aSO_2$-, wobei $R^a$ die oben angegebene Bedeutung hat,

$R^3$ H, Halogen, $C_1$-$C_2$-Alkyl, $C_1$-$C_2$-Halogenalkyl, $C_1$-$C_2$-Alkoxy, $C_1$-$C_2$-Halogenalkoxy, $C_1$-$C_2$-Alkylthio und

$R^4$ $C_1$-$C_3$-Halogenalkyl,

bedeuten.

**3.** Verwendung nach Anspruch 1 oder 2, dadurch gekennzeichnet, daß

n 0 bis 3;

$R^1$ $C_1$-$C_3$-Alkyl, Cyclopentyl oder Phenyl;

$R^2$ Fluor, Chlor, $NO_2$, CN, Methyl, Trifluormethyl, Methoxy, Difluormethoxy, Trifluormethoxy oder Methylsulfonyl;

$R^3$ Wasserstoff, Halogen, Methyl, $CF_3$, Methoxy, Difluormethoxy, Trifluormethoxy oder Methylthio, und

$R^4$ $CH_2F$, $CHF_2$, $CH_2Cl$, $CHCl_2$, $CCl_3$, $CH_2CH_2Cl$, $CH_2CH_2F$, C $H_2CHF_2$, $CH_2CF_3$, $CHFCH_3$, $CHFCH_2F$, $CHFCHF_2$, $CF_2CH_3$, $CF_2CH_2F$, $CF_2CHF_2$, $CF_2CF_3$, $CF_2CHFCl$, $CHFCHFCl$, $CH_2CCl_3$, $CH_2CHCl_2$, $CHFCHCl_2$, $CF_2CHCl_2$, $CHFCH_2Cl$ oder $CF_2CH_2Cl$ bedeuten.

**4.** Verfahren zur Herstellung von Verbindungen der in Anspruch 1 definierten Formel (I) oder deren Salzen, dadurch gekennzeichnet, daß man

a) ein Cyclohexandion der Formel (II)

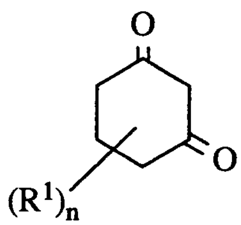

(II)

mit einem Säurechlorid der Formel (III)

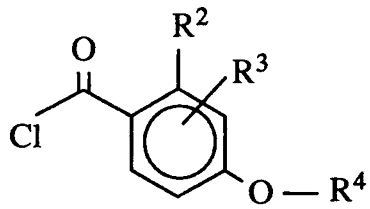

(III)

zu einem Enolether der Formel (IV)

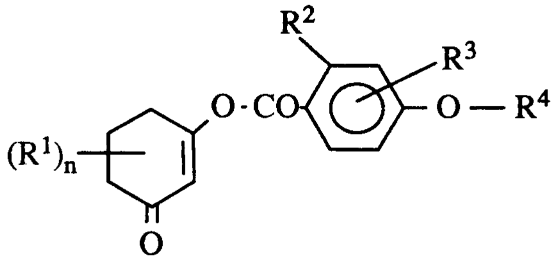

(IV)

umsetzt und diesen in Gegenwart einer Cyanid-Quelle oder einer Lewis-Säure zu einer Verbindung der genannten Formel (I) umlagert

oder

b) ein Cyclohexandion der genannten Formel (II) mit einem Benzoylcyanid der Formel (V)

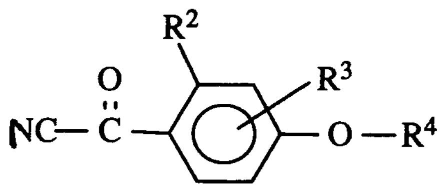

(V)

umsetzt, wobei in den Formeln (II) bis (V) $R^1$, $R^2$, $R^3$, $R^4$ und n die in Formel (I) genannte Bedeutung haben.

**5.** Verfahren zur Herstellung eines herbiziden oder pflanzenwachstumsregulatorischen Mittels, dadurch gekennzeichnet, daß man eine Verbindung der in Anspruch 1, 2 oder 3 definierten Formel (I) oder deren Salze mit Formulierungshilfsmittel nach üblichen Methoden zu einer Pflanzenschutzformulierung verarbeitet.

**6.** Verwendung von Verbindungen der in Anspruch 1, 2 oder 3 definierten Formel (I) oder deren Salzen, als Pflanzenwachstumsregulatoren.

**7.** Verfahren zur selektiven Bekämpfung von Schadpflanzen, dadurch gekennzeichnet, daß man eine wirksame Menge einer in einem der Ansprüche 1 bis 3 definierten Verbindungen der Formel I oder von deren Salzen auf die Pflanzen, Pflanzensamen oder die Anbaufläche appliziert.

**8.** Verfahren zur Wachstumsregulierung von Pflanzen, dadurch gekennzeichnet, daß man eine wirksame Menge einer in einem der Ansprüche 1 bis 3 definierten Verbindungen der Formel I oder von deren Salzen auf die Pflanzen, Pflanzensamen oder die Anbaufläche appliziert.

**9.** Verwendung von Verbindungen der Formel (VIII)

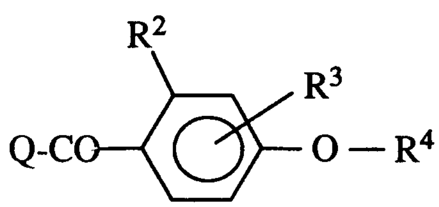

(VIII)

worin Q Cl, CN, OH oder $O^-Me^+$, wobei $Me^+$ ein anorganisches oder organisches Kation ist, bedeutet, und $R^2$, $R^3$ und $R^4$ wie in Formel (I) nach einem der Ansprüche 1 bis 3 definiert sind, zur Herstellung von Verbindungen der Formel (I) oder deren Salze.

**10.** Verfahren zur Herstellung der in Anspruch 9 definierten Verbindungen der Formel VIII, dadurch gekennzeichnet, daß man entsprechende Toluole der Formel (VII)

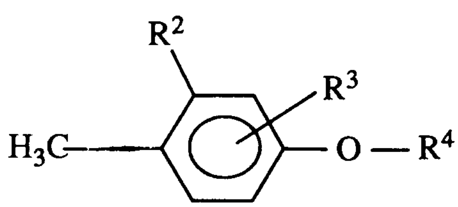

(VII)

mit Kaliumpermanganat oder Salpersäure zu Verbindungen der Formel VIII, worin Q = COOH bedeutet, oxidiert und, im Fall Q = Cl, halogeniert oder, im Falle Q = CN, in das Benzoylcyanid

umwandelt.